# FASCICULE DE BREVET EUROPEEN

(11) **EP 0 932 421 B1**
(45) Date de publication et mention de la délivrance du brevet: **18.04.2001**
(21) Numéro de dépôt: 97940206.2
(22) Date de dépôt: 10.09.1997
(51) Int. Cl.: A61M 1/00

(54) **DISPOSITIF D'ASPIRATION OU D'INSUFFLATION A USAGE MEDICAL, COMPRENANT UNE VALVE D'ARRET**
MEDIZINISCHE ASPIRATIONS- ODER INSUFFLATIONSVORRICHTUNG MIT ABSPERRVENTIL
SUCKING OR INSUFFLATING DEVICE FOR MEDICAL USE, COMPRISING A STOP VALVE

(30) Priorité: 13.09.1996 FR 9611397
(43) Date de publication de la demande: 04.08.1999
(73) Titulaire: CAIR L.G.L., 69380 Civrieux d'Azergues (FR)
(72) Inventeur: LOPEZ, Georges, Antoine, F-69290 Craponne (FR)
(74) Mandataire: Vuillermoz, Bruno
(86) Numéro de dépôt international: FR9701591
(87) Numéro de publication internationale: WO9810809

(56) Documents cités:
- FR-A- 2 365 157

## Description

La présente invention concerne un dispositif d'aspiration ou d'insufflation à usage médical, comprenant une valve d'arrêt.

Un tel dispositif est notamment destiné à permettre l'aspiration de liquides corporels, tels que la salive ou les sécrétions broncho-pulmonaires, ou de liquides chirurgicaux lors d'interventions chirurgicales.

Pour réaliser une aspiration de salive ou de sécrétions broncho-pulmonaires, le robinet mural d'aspiration que comprend la chambre d'hôpital est relié par un tuyau à un récipient de collecte des sécrétions, et ce récipient est relié à un tuyau muni, à son extrémité libre, d'une canule d'aspiration et d'une valve d'arrêt permettant le contrôle de l'aspiration sans manoeuvre du robinet mural.

Selon une technique, cette valve consiste en une pince métallique à usages multiples, dont les mors viennent écraser la paroi du tuyau.

Cette pince a pour inconvénient d'être lourde, de n'être utilisable qu'avec des tuyaux souples à mémoire de forme, du type en silicone, qui sont onéreux, d'être parfois difficile à manipuler pour le personnel médical et impossible à manipuler par le patient lui-même, et d'impliquer un risque de contamination.

Une autre technique consiste à prévoir un orifice sur l'arrière de la canule, débouchant dans l'intérieur du conduit de la canule. Cet orifice peut être obturé manuellement pour permettre l'aspiration.

Un tel dispositif est à usage unique, est léger, simple d'utilisation et a un coût modéré, mais rend possible un contact des sécrétions aspirées avec les doigts de l'utilisateur, et génère un sifflement impliquant un arrêt de l'aspiration en période de non fonctionnement, par manoeuvre du robinet mural.

En outre, la généralisation de ce type de dispositif dans un hôpital peut aboutir à une perte de charge importante au niveau du réseau d'aspiration.

D'autres valves comprennent un raccord pourvu d'un orifice susceptible d'être obturé par le doigt de l'utilisateur, et d'une membrane souple dont le déplacement commande l'aspiration, le déplacement de cette membrane étant provoqué par la fermeture de l'orifice.

Ces dispositifs ont sensiblement les mêmes inconvénients que ceux indiqués ci-dessus, et présentent en outre un risque d'arrêt de fonctionnement par collage de la membrane sur son siège en cas de non utilisation prolongée sans rinçage de la valve.

Une autre technique consiste à prévoir une valve comportant un bouton-poussoir d'actionnement d'un organe d'obturation.

Une telle valve a pour inconvénient d'avoir un prix de revient élevé du fait de la pluralité de pièces qu'elle comprend et de l'assemblage que celles-ci impliquent. De plus, les patients affaiblis peuvent ne pas avoir la force nécessaire à l'actionnement du bouton-poussoir, et il existe un certain risque de déconnexion de la valve en cours d'utilisation.

Une autre technique encore consiste à assembler un tuyau souple, du type en silicone, à une pièce en matière plastique de forme cylindrique, comprenant des orifices, cette pièce permettant de commander l'aspiration par déformation manuelle de la paroi du tuyau.

Ce dispositif a pour inconvénient d'être encombrant, d'avoir un prix élevé compte tenu de la nécessité d'utiliser un tuyau en silicone, d'avoir une zone à déformer difficile à localiser, impliquant un risque majeur de non aspiration en cas d'urgence, et de présenter un risque de déconnexion entre ladite pièce cylindrique et la canule d'aspiration.

Le document FR-A-2 365 157 décrit un dispositif d'aspiration constitué d'un corps central sur lequel porte une membrane tubulaire. Une enveloppe extérieure entoure l'ensemble corps central et membrane tubulaire délimitant ainsi une chambre de commande. Un évent est percé dans l'enveloppe, de sorte que la chambre de commande se trouve à l'atmosphère aussi longtemps que l'évent n'est pas obturé, l'obturation étant effectuée par le doigts de l'utilisateur.

Ce dispositif présente l'inconvénient d'avoir une zone difficile à localiser, impliquant un risque majeur de non aspiration en cas d'urgence.

La présente invention vise à remédier à ces différents inconvénients.

Pour ce faire, elle propose un dispositif d'aspiration ou d'insufflation à usage médical, destiné à être relié à un tuyau et comprenant une valve d'arrêt et une partie tubulaire destinée à être emmanchée dans le tuyau, cette partie comprenant :
- une paroi transversale interrompant le conduit qu'elle délimite ;
- des orifices traversant la paroi tubulaire, dont au moins un est situé d'un côté de la paroi transversale précitée, tandis qu'au moins un autre est situé de l'autre côté de cette même paroi, au niveau des évidements, le ou les orifices situés sur au moins un côté de la paroi transversale étant normalement recouverts, avec étanchéité, par la paroi du tuyau et ;
- au moins un évidement permettant de déformer la paroi du tuyau de manière à éloigner celle-ci d'au moins un orifice, afin de rendre le passage du flux d'air possible au travers des différents orifices précités, l'axe transversal des orifices étant parallèle à la surface de l'évidement ;
**caractérisé** en ce que deux leviers pivotants viennent appuyer, dans une position de pivotement, sur le tuyau au niveau de l'évidement pour réaliser la déformation de ce tuyau.

Le dispositif selon l'invention permet ainsi de commander l'aspiration ou l'arrêt de cette aspiration en manoeuvrant le levier pour déformer la paroi du tuyau.

Ce levier permet à un patient, même affaibli, de pouvoir parfaitement et immédiatement manipuler la valve, en fonction de ses besoins.

Ce levier sert également au maintien manuel de la sonde d'aspiration, de sorte que le patient n'a qu'à appuyer sur le levier pour déclencher l'aspiration.

De plus, grâce à ce levier, le dispositif selon l'invention peut être employé avec un tuyau de souplesse moyenne, ce qui évite l'emploi de tuyaux très souples, du type silicone, relativement onéreux.

Le levier permet également l'accrochage du dispositif sur un rail de support.

L'invention fournit ainsi un dispositif à usage unique, éliminant tout risque de contact direct avec les sécrétions aspirées, ne produisant aucun sifflement en position d'arrêt de l'aspiration, et ayant un fonctionnement très fiable.

De préférence, le dispositif comprend deux évidements situés de part et d'autre de la partie tubulaire et deux leviers latéraux situés en regard de ces évidements.

Ces deux leviers, situés de manière diamètralement opposée l'un par rapport à l'autre, permettent une parfaite déformation de la paroi du tuyau et une parfaite préhension et manipulation du dispositif.

Chaque levier comprend avantageusement une saillie à arête vive permettant de venir presser la paroi du tuyau contre la paroi délimitant le fond de l'évidement. Cette saillie assure ainsi un bon écartement de la paroi du tuyau par rapport à ou aux orifice(s).

Avantageusement, le dispositif est réalisé par moulage d'une même pièce de matière synthétique, le mouvement des leviers étant rendu possible par déformation et rappel élastique de cette matière synthétique. Le coût de fabrication du dispositif est ainsi très limité.

Dans ce cas, le dispositif comprend avantageusement une canule formant corps avec lui-même, celle-ci étant de préférence moulée en une seule pièce avec lui. Cette intégration de la canule au dispositif élimine les risques de déconnexion accidentelle entre la canule et la valve.

De préférence, la matière constituant le dispositif est transparente, ce qui permet de visualiser les sécrétions aspirées et d'apprécier l'encombrement du canal d'aspiration.

Pour sa bonne compréhension, l'invention est à nouveau décrite ci-dessous en référence au dessin schématique annexé représentant, à titre d'exemple non limitatif, une forme de réalisation préférée du dispositif qu'elle concerne.
La figure 1 en est une vue en perspective, avant raccordement à l'extrémité d'un tuyau d'aspiration ;
la figure 2 en est une vue de profil, après raccordement à ce tuyau, ce dernier étant coupé longitudinalement ;
la figure 3 en est une vue en coupe selon la ligne III-III de la figure 2 et à échelle agrandie, et
la figure 4 en est une vue similaire à la figure 3, selon une autre position de fonctionnement, correspondant à l'ouverture de la valve que comprend ce dispositif.

Les figures représentent, sous différents angles, un dispositif 1 à usage médical, permettant l'aspiration de salive ou de sécrétions broncho-pulmonaires.

Ce dispositif 1 est destiné à être relié à une extrémité d'un tuyau d'aspiration 2, dont l'autre extrémité est reliée à un récipient de collecte des sécrétions (non représenté). Ce récipient est relié par un autre tuyau à un robinet mural d'aspiration que comprend la chambre d'hôpital.

Le dispositif 1 est réalisé par moulage d'une même pièce de matière synthétique transparente, présentant des caractéristiques de déformabilité élastique explicitées plus loin. Il comprend :
- une partie tubulaire 5 destinée à être emmanchée dans le tuyau 2, délimitant un conduit 6 ;
- deux leviers pivotants 7 situés de part et d'autre de cette partie tubulaire 5, en étant positionnés de manière diamétralement opposée l'un par rapport à l'autre, et
- une canule d'aspiration 8 coaxiale à la partie tubulaire 5.

Cette dernière comprend :
- une paroi transversale 10 interrompant le conduit 6,
- deux orifices radiaux 11 traversant sa paroi, situés entre la paroi transversale 10 et l'extrémité de la partie 5 la plus profondément engagée dans le tuyau 2 ; ces orifices 11 sont disposés de manière diamétralement opposés l'un par rapport à l'autre, selon un axe perpendiculaire à celui sur lequel sont placés les leviers 7,
- une partie 5a, amincie latéralement, qui délimite deux évidements latéraux 12, aménagés en regard des leviers 7, et
- deux orifices 13 aménagés de manière diamétralement opposés l'un par rapport à l'autre, perpendiculairement à l'axe des orifices 11, de l'autre côté de la paroi transversale 10 par rapport à ces orifices 11 ; les orifices 13 débouchent dans les évidements 12 d'une part et dans le conduit 15 de la canule 8 d'autre part.

Les leviers 7 sont reliés, au niveau de leur base, à une collerette 16 raccordant la partie tubulaire 5 et la canule 8.

Chacun d'eux comprend une saillie 18 à arête vive, aménagée à hauteur des orifices 11, ainsi qu'une extrémité libre élargie 19 formant une surface d'appui manuel.

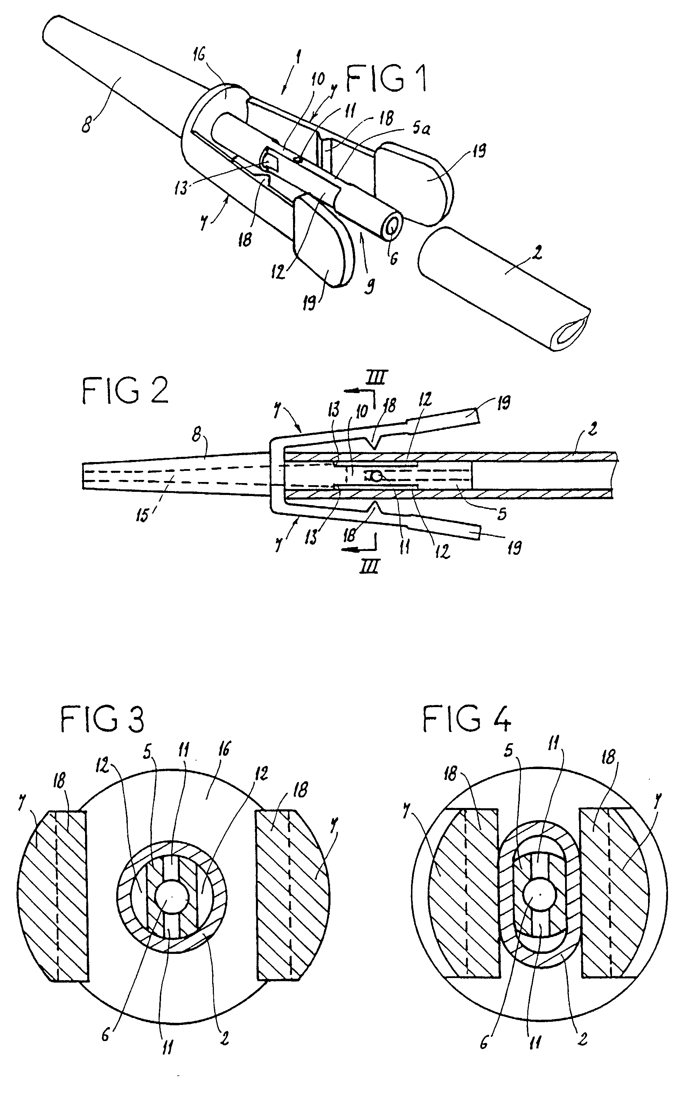

La figure 3 montre que les orifices 11 sont normalement recouverts, avec étanchéité, par la paroi du tuyau 2 lorsque la partie tubulaire 5 est emmanchée dans l'extrémité de celui-ci.

Chaque levier 7 forme normalement un angle de l'ordre de 10° par rapport à l'axe longitudinal du dispositif 1, de sorte que leur saillie 18 se trouve, dans cette position, à distance de la paroi du tuyau 2, ainsi que le montrent les figures 2 et 3.

Le degré de souplesse élastique de la matière constituant le dispositif 1 est tel que les leviers 7 peuvent être déformés manuellement entre la position normale, montrée aux figures 2 et 3, et la position montrée à la figure 4, dans laquelle leurs saillies 18 viennent appuyer la paroi du tuyau 2 contre les parois de la partie tubulaire 5 délimitant les fonds des évidements 12.

Cette déformation permet de parfaitement ovaliser le tuyau 2 et, donc, d'éloigner suffisamment la paroi de ce tuyau 2 des orifices 11 pour assurer le passage de l'air successivement au travers du conduit 6, des orifices 11, des évidements 12, des orifices 13 et du conduit 15. Dès que la pression est relâchée sur les leviers 7, le tuyau 2 retrouve sa forme d'origine, interrompant immédiatement l'écoulement du flux d'air.

L'invention fournit ainsi un dispositif d'aspiration ou d'insufflation perfectionné, présentant de nombreux avantages par rapport aux dispositifs de la technique antérieure. Il permet notamment à un patient, même affaibli, de pouvoir parfaitement et immédiatement commander l'aspiration ou l'insufflation en fonction de ses besoins, et peut être employé avec un tuyau 2 de souplesse moyenne, ce qui évite l'emploi de tuyaux très souples, du type silicone.

Il va de soi que l'invention n'est pas limitée à la forme de réalisation décrite ci-dessus à titre d'exemple mais qu'elle en embrasse, au contraire, toutes les variantes de réalisation. Ainsi, le dispositif pourrait être réalisé en plusieurs pièces assemblées. Par exemple, le ou les leviers peuvent être constitués par des pièces distinctes du corps du dispositif, et être montés pivotants sur celui-ci au moyen d'axes métalliques. Le ou les leviers pourraient également être solidaires d'un oeillet pouvant être engagé sur le corps du dispositif et être retenu sur celui-ci, par exemple par coincement.

## Revendications

1. Dispositif d'aspiration ou d'insufflation à usage médical, destiné à être relié à un tuyau (2) et comprenant une valve d'arrêt et une partie tubulaire (5) destinée à être emmanchée dans le tuyau (2), cette partie (5) comprenant :
- une paroi transversale (10) interrompant le conduit (6) qu'elle délimite ;
- des orifices (11) traversant la paroi tubulaire (5), dont au moins un est situé d'un côté de la paroi transversale (10) précitée, tandis qu'au moins un autre (13) est situé de l'autre côté de cette même paroi (10), au niveau des évidements (12), le ou les orifices (11) situés sur au moins un côté de la paroi transversale (10) étant normalement recouverts, avec étanchéité, par la paroi du tuyau (2), et ;
- au moins un évidement (12) permettant de déformer la paroi du tuyau (2) de manière à éloigner celle-ci d'au moins un orifice (11), afin de rendre le passage du flux d'air possible au travers des différents orifices (11,13) précités, l'axe transversal des orifices (11) étant parallèle à la surface de l'évidement (12);
**caractérisé** en ce que deux leviers pivotants viennent appuyer, dans une position de pivotement, sur le tuyau (2) au niveau de l'évidement (12) pour réaliser la déformation de ce tuyau (2).

2. Dispositif selon la revendication 1, caractérisé en ce qu'il comprend deux évidements (12) situés de part et d'autre de la partie tubulaire (5) et deux leviers latéraux (7) situés en regard de ces évidements (12).

3. Dispositif selon la revendication 1 ou 2, caractérisé en ce que chaque levier (7) comprend une saillie (18) à arête vive venant presser la paroi du tuyau (2) contre la paroi délimitant le fond de l'évidement (12).

4. Dispositif selon l'une des revendications 1 à 3, caractérisé en ce qu'il est réalisé par moulage d'une même pièce de matière synthétique, le mouvement des leviers (7) étant rendu possible par déformation et rappel élastique de cette matière synthétique.

5. Dispositif selon l'une des revendications 1 à 4, caractérisé en ce qu'il comprend une canule (8) formant corps avec lui-même.

6. Dispositif selon l'une des revendications 1 à 5, caractérisé en ce que la matière qui le constitue est transparente.

## Claims

1. Aspiration or insufflation device for medical use, intended to be connected to a tubing (2) and comprising a stop valve and a tubular part (5) which is intended to be fitted into the tubing (2), this part (5) comprising:
- a transverse wall (10) interrupting the conduit (6) which it delimits;
- orifices (11) passing through the tubular wall (5), of which at least one is situated on one side of the aforementioned transverse wall (10), while at least one other (13) is situated on the other side of this same wall (10), in the area of the recesses (12), the orifice or orifices (11) situated on at least one side of the transverse wall (10) being normally covered, sealingly, by the wall of the tubing (2), and;
- at least one recess (12) permitting deformation of the wall of the tubing (2) in order to move the latter away from at least one orifice (11), so as to enable the flow of air to pass through the various aforementioned orifices (11, 13), the transverse axis of the orifices (11) being parallel with the surface of the recess (12);
characterized in that two pivoting levers come to bear, in a pivoting position, on the tubing (2) in the area of the recess (12) in order to deform this tubing (2).

2. Device according to Claim 1, characterized in that it comprises two recesses (12) situated on each side of the tubular part (5) and two lateral levers (7) situated opposite these recesses (12).

3. Device according to Claim 1 or 2, characterized in that each lever (7) comprises a sharp-edged projection (18) which presses the wall of the tubing (2) against the wall delimiting the bottom of the recess (12).

4. Device according to one of Claims 1 to 3, characterized in that it is produced by moulding of one and the same piece of synthetic material, the movement of the levers (7) being made possible by deformation and elastic return of this synthetic material.

5. Device according to one of Claims 1 to 4, characterized in that it comprises a cannula (8) made integral with it.

6. Device according to one of Claims 1 to 5, characterized in that the material from which it is made is transparent.

## Patentansprüche

1. Medizinische Aspirations- oder Insufflationsvorrichtung zur Verbindung mit einem Schlauch (2), welche ein Absperrventil und einen rohrförmigen Abschnitt (5) aufweist, der zum Einstecken in den Schlauch (2) vorgesehen ist, wobei der Abschnitt (5) folgendes umfaßt:
- eine Querwandung (10), welche den Kanal (6), den sie begrenzt, unterbricht;
- Öffnungen (11), welche die rohrförmige Wandung (5) durchbrechen, von denen wenigstens eine auf einer Seite der genannten Querwandung (10) und mindestens eine weitere (13) auf der anderen Seite dieser Wandung (10) im Bereich von Aussparungen (12) angeordnet ist, wobei die auf mindestens einer Seite der Querwandung (10) angeordnete(n) Öffnung oder Öffnungen (11) normalerweise dicht durch die Wandung des Schlauches (2) abgedeckt ist/sind, und;
- mindestens eine Aussparung (12), die die Verformung der Wandung des Schlauches (2) ermöglicht, um diese von der mindestens einen Öffnung (11) zu entfernen, um den Durchtritt eines Luftstroms durch die verschiedenen genannten Öffnungen (11, 13) zu ermöglichen, wobei die Querachse der Öffnungen (11) parallel zur Oberfläche der Aussparung (12) verläuft;
**dadurch gekennzeichnet**, daß zwei Schwenkhebel sich in einer Schwenkposition auf den Schlauch (2) im Bereich der Aussparung (12) abstützen, um die Verformung dieses Schlauches (2) zu bewirken.

2. Vorrichtung nach Anspruch 1, **dadurch gekennzeichnet**, daß sie zwei Aussparungen (12) aufweist, die auf zwei Seiten des rohrförmigen Abschnitts (5) angeordnet sind, und daß sie zwei seitliche Hebel (7) aufweist, die gegenüber diesen Aussparungen (12) angeordnet sind.

3. Vorrichtung nach Anspruch 1 oder 2, **dadurch gekennzeichnet**, daß jeder Hebel (7) einen scharfkantigen Vorsprung (18) aufweist, der die Wandung des Schlauches (2) gegen die den Grund der Aussparung (12) begrenzende Wandung drückt.

4. Vorrichtung nach einem der Ansprüche 1 bis 3, **dadurch gekennzeichnet**, daß sie durch Gießen aus einem einzigen Stück aus synthetischem Material hergestellt ist, wobei die Bewegung der Hebel (7) durch Verformung und elastische Rückstellung dieses synthetischen Materials ermöglicht wird.

5. Vorrichtung nach einem der Ansprüche 1 bis 4, **dadurch gekennzeichnet**, daß sie eine einstückig angeformte Kanüle (8) aufweist.

6. Vorrichtung nach einem der Ansprüche 1 bis 5, **dadurch gekennzeichnet**, daß das Material, aus dem sie gebildet ist, transparent ist.
